# EUROPEAN PATENT APPLICATION

(11) **EP 4 775 672 A1**
(43) Date of publication of application: **15.07.2026**
(21) Application number: 24862817.4
(22) Date of filing: 04.09.2024
(51) Int. Cl.: C12N 7/01, C12N 5/00, C12N 15/09

(54) **CULTURE MEDIUM COMPOSITION**

(30) Priority: 05.09.2023 JP 2023143946
(71) Applicant: AJINOMOTO CO., INC., Chuo-ku Tokyo 104-8315 (JP)
(72) Inventor: HIGUCHI, Takuya, Kawasaki-shi, Kanagawa 210-8681 (JP)
(74) Representative: Hoffmann Eitle
(86) International application number: PCT/JP2024/031677
(87) International publication number: WO 2025/053165

(57) **Abstract**

The present invention provides a medium composition containing a pyruvate dehydrogenase kinase inhibitor for promoting production of a virus carrying a gene of interest, when culturing virus-producing cells into which the gene of interest has been introduced, a medium composition for promoting production of virus or virus-like particles when culturing cells that produce the virus or virus-like particles, and the like.

## Description

### [Technical Field]

The present invention relates to a medium composition containing a pyruvate dehydrogenase kinase inhibitor for promoting production of a virus carrying a gene of interest, when culturing virus-producing cells into which the gene of interest has been introduced, and for promoting production of virus or virus-like particles when culturing cells that produce the virus or virus-like particles.

### [Background Art]

In order to meet the needs for human gene therapy, vaccine production, and the like, techniques for rapidly and stably producing viruses and the like are essential. Particularly, culturing of cells that produce viruses and the like (hereinafter also to be referred to as virus-producing cells) is extremely important.

When designing and optimizing a medium for culturing virus-producing cells, the components of the medium pose an important problem. Components contained in the medium affect the growth and proliferation of the cells. For example, Patent Literature 1 discloses that the culture efficiency of animal cells is improved by culturing the cells in a medium containing a pyruvate dehydrogenase kinase inhibitor.

In addition, in the case of media for culturing virus-producing cells, the components contained in the media also affect the virus production ability within the cells. Furthermore, when producing viruses by using cells transfected with a gene that produces a cancer-suppressing protein, for example, the components contained in the media also affect the proportion of viruses carrying said gene in the entire viruses produced. However, the components known to date have not necessarily produced satisfactory results. For example, when producing adeno-associated viruses by using cells transfected with a gene, the proportion of adeno-associated viruses carrying said gene is said to be about 5% to 30% of the adeno-associated viruses (Non Patent Literature 1).

### [Citation List]

### [Patent Literature]

[Patent Literature 1]
WO 2002/04598

### [Non Patent Literature]

[Non Patent Literature 1]
Molecular Therapy Methods & Clinical Development; 2021 June 11; Vol.21; p.642-655

### [Summary of Invention]

### [Technical Problem]

The present invention aims to provide a medium composition for improving the production yield of viruses carrying a gene of interest when producing viruses carrying the gene of interest by using virus-producing cells, and one for promoting the production of viruses or virus-like particles when producing the viruses or virus-like particles by using cells, and the like.

### [Solution to Problem]

The present inventors have conducted intensive studies of the above-mentioned problem and found that the ability to produce an adeno-associated virus carrying a gene of interest is improved when sodium dichloroacetate, which is a a pyruvate dehydrogenase kinase inhibitor, is added to human embryonic kidney cell 293 (HEK293). Based on such findings, they have conducted further studies and completed the present invention.

Accordingly, the present invention provides the following.
[1] A medium composition for promoting production of a virus carrying a gene of interest for culturing a virus-producing cell into which the gene of interest has been introduced, the medium composition comprising a pyruvate dehydrogenase kinase inhibitor.
[2] The medium composition of the above-mentioned [1], wherein the pyruvate dehydrogenase kinase inhibitor is dichloroacetic acid or a salt thereof.
[3] The medium composition of the above-mentioned [1] or [2], wherein the cell is a cell selected from the group consisting of a HEK293 cell, an MDCK cell, a Vero cell, an A549 cell, a PerC6 cell, a HeLa cell, and an insect cell.
[4] The medium composition of the above-mentioned [1] or [2], wherein the cell is an HEK293 cell.
[5] The medium composition of any of the above-mentioned [1] to [4] wherein the virus-producing cell is a cell that produces adeno-associated virus.
[6] The medium composition of any of the above-mentioned [1] to [5], wherein a concentration of the pyruvate dehydrogenase kinase inhibitor in the medium composition is 0.1 mM to 1,000 mM.
[7] A medium additive for promoting production of a virus carrying a gene of interest, when culturing a virus-producing cell into which the gene of interest has been introduced, the medium additive comprising a pyruvate dehydrogenase kinase inhibitor.
[8] Use of a pyruvate dehydrogenase kinase inhibitor in the manufacture of a medium composition for promoting production of a virus carrying a gene of interest, when culturing a virus-producing cell into which the gene of interest has been introduced.
[9] A method for promoting production of a virus carrying a gene of interest, comprising a step of culturing a virus-producing cell into which the gene of interest has been introduced, in a medium comprising a pyruvate dehydrogenase kinase inhibitor.
[10] A method for producing a virus carrying a gene of interest, comprising a step of culturing a virus-producing cell into which the gene of interest has been introduced, in a medium comprising a pyruvate dehydrogenase kinase inhibitor.
[11] A medium composition for promoting production of a virus or virus-like particle when culturing a cell that produces the virus or virus-like particle, the medium composition comprising a pyruvate dehydrogenase kinase inhibitor.
[12] The medium composition of the above-mentioned [11], wherein the pyruvate dehydrogenase kinase inhibitor is dichloroacetic acid or a salt thereof.
[13] The medium composition of the above-mentioned [11] or [12], wherein the cell is a cell selected from the group consisting of a HEK293 cell, an MDCK cell, a Vero cell, an A549 cell, a PerC6 cell, an HeLa cell, and an insect cell.
[14] The medium composition of the above-mentioned [11] or [12], wherein the cell is an HEK293 cell.
[15] The medium composition of any of the above-mentioned [11] to [14], wherein the cell that produces the virus or virus-like particle is a cell that produces adeno-associated virus.
[16] The medium composition of any of the above-mentioned [11] to [15], wherein a concentration of the pyruvate dehydrogenase kinase inhibitor in the medium composition is 0.1 mM to 1,000 mM.
[17] A medium additive for promoting production of a virus or virus-like particle when culturing a cell that produces the virus or virus-like particle, the medium additive comprising a pyruvate dehydrogenase kinase inhibitor.
[18] Use of a pyruvate dehydrogenase kinase inhibitor in the manufacture of a medium composition for promoting production of a virus or virus-like particle, when culturing a cell that produces the virus or virus-like particle.
[19] A method for promoting production of a virus or virus-like particle, comprising a step of culturing a cell that produces the virus or virus-like particle in a medium comprising a pyruvate dehydrogenase kinase inhibitor.
[20] A method for producing a virus or virus-like particle, comprising a step of culturing a cell that produces the virus or virus-like particle in a medium comprising a pyruvate dehydrogenase kinase inhibitor.

### [Advantageous Effects of Invention]

The present invention makes it possible to improve the production yield of a virus carrying a gene of interest when producing the virus carrying the gene of interest using virus-producing cells, and the like.

### [Brief Description of Drawings]

[Fig. 1]

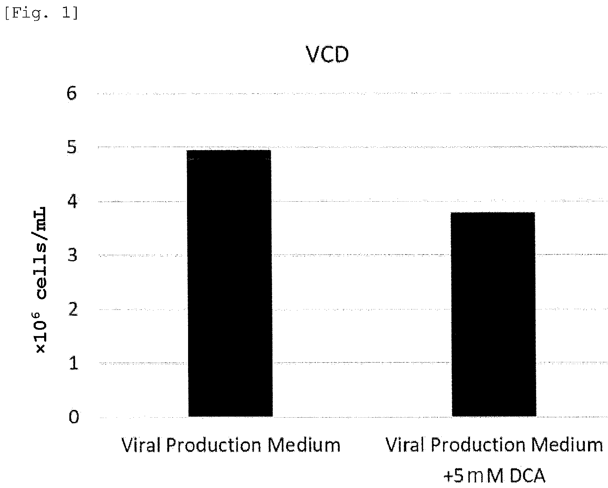

   Fig. 1 shows the verification results of the effect of sodium dichloroacetate on the proliferation of HEK293 in Example 1. VCD means viable cell density, and the vertical axis shows viable cell density (×10⁶ cells/mL). The "Viral Production Medium" on the horizontal axis is the case where a medium containing no sodium dichloroacetate was used, and the "Viral Production Medium + 5 mM DCA" is the case where a medium containing sodium dichloroacetate at a final concentration of 5 mM was used.
[Fig. 2]

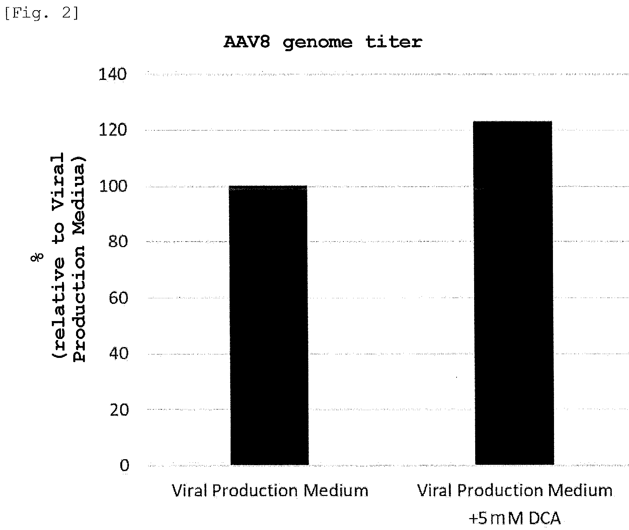

   Fig. 2 shows the verification results of the effect of sodium dichloroacetate on the AAV8 genome titer in Example 1. The vertical axis shows the relative AAV8 genome titer, where the AAV8 genome titer obtained when AAV8 is produced using a medium without sodium dichloroacetate is 100%. The "Viral Production Medium" on the horizontal axis is the case where AAV8 was produced using a medium without sodium dichloroacetate, and the "Viral Production Medium + 5 mM DCA" is the case where AAV8 was produced using a medium containing sodium dichloroacetate at a final concentration of 5 mM.
[Fig. 3]

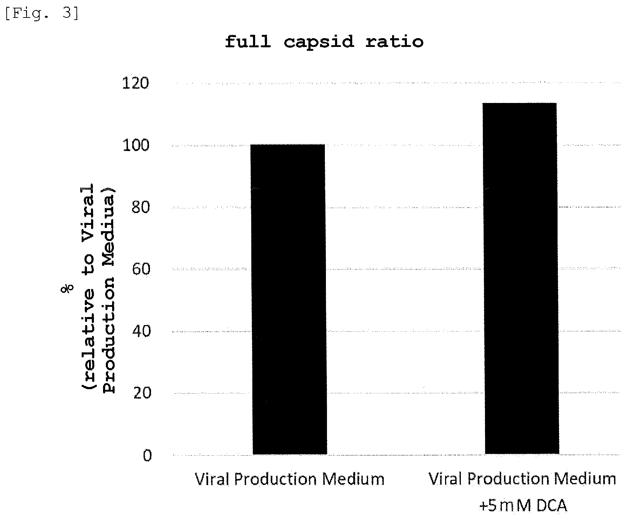

   Fig. 3 shows the verification results of the effect of sodium dichloroacetate on the full/empty capsid ratio (full capsid ratio) in Example 1. The vertical axis shows the relative full capsid ratio, where the full capsid ratio obtained when AAV8 is produced using a medium without sodium dichloroacetate is 100%. The "Viral Production Medium" on the horizontal axis is the case where AAV8 was produced using a medium without sodium dichloroacetate, and the "Viral Production Medium + 5 mM DCA" is the case where AAV8 was produced using a medium containing sodium dichloroacetate at a final concentration of 5 mM.

### [Description of Embodiments]

### 1. Medium composition 1

The present invention provides a medium composition for promoting production of a virus carrying a gene of interest, when culturing a virus-producing cell into which the gene of interest has been introduced, the medium composition containing a pyruvate dehydrogenase kinase inhibitor (hereinafter also to be referred to as "the medium composition 1 of the present invention").

Pyruvate dehydrogenase (PDH) kinase is an enzyme that phosphorylates and inactivates PDH, and regulates activity in an antagonistic manner with PDH phosphatase. A PDH kinase inhibitor is an organic molecule that prevents PDH kinase from phosphorylating a specific serine residue on E1 (alpha subunit) of a PDH complex, which is involved in regulating PDH activity.

As the PDH kinase inhibitor, various compounds are known, including dichloroacetic acid or a salt thereof, AZD7545, VER-246608, PDHK-IN-5, Relamin, and the like, and dichloroacetic acid or a salt thereof is preferred.

The salt of dichloroacetic acid includes metal salt, ammonium salt, organic amine addition salt, and the like.

Metal salts include alkali metal salts such as sodium salt, potassium salt, and the like, alkaline earth metal salts such as magnesium salt, calcium salt, and the like, aluminum salt, zinc salt, and the like. Ammonium salts include salts such as ammonium salt, diisopropylammonium salt, tetramethylammonium salt, and the like. Organic amine addition salts include addition salts of morpholine, piperidine, or the like. Among these, sodium salts and potassium salts are preferred, and sodium salts are more preferred.

When obtaining a salt of dichloroacetic acid and dichloroacetic acid is obtained in the form of a salt, it can be purified as is. When dichloroacetic acid is obtained in a free form, it can be isolated and purified by dissolving or suspending dichloroacetic acid in an appropriate solvent and adding an acid or base.

Dichloroacetic acid or a salt thereof may also exist in the form of a solvate such as hydrate or the like. In the present invention, these solvates are also referred to as dichloroacetic acid or a salt thereof.

The PDH kinase inhibitor used in the medium composition 1 of the present invention may be synthesized by a known method or can also be obtained as a commercially available product.

The concentration of the PDH kinase inhibitor in the medium composition 1 of the present invention is not particularly limited as long as the effects of the present invention are achieved, and is generally 0.1 mM to 1,000 mM, preferably 0.3 mM to 300 mM, more preferably 0.5 mM to 100 mM, further preferably 1 mM to 20 mM.

In the present specification, "gene of interest" refers to a heterologous polynucleotide that can be introduced into a cell or organism via a viral vector or the like. It can be any polynucleotide, such as a gene encoding a polypeptide or protein, or a polynucleotide that can be transcribed into an inhibitory polynucleotide (e.g., siRNA, miRNA, shRNA, etc.), and is preferably intended for the treatment or the like of diseases in animals including humans.

Examples of the virus include adenovirus, adeno-associated virus (AAV), retrovirus, lentivirus, and Sendai virus, as well as orthomyxovirus, paramyxovirus, reovirus, picornavirus, flavivirus, arenavirus, herpesvirus, and poxvirus, as well as recombinant viruses of these. Among these, adenovirus and AAV are preferred, and AAV is more preferred. Examples of AAV include AAV1, AAV2, AAV3, AAV4, AAV5, AAV6, AAV7, AAV8, AAV9, AAV10, AAV11, AAV12, AAV13, AAVDJ, AAVDJ8, AAV-B1, AAVM41, AAVrhlO, AAVrh74, and chimeras thereof. AAV1, AAV2, AAV4, AAV5, AAV6, AAV7, AAV8, and AAV9 are more preferred, and AAV2 and AAV8 are further preferred.

The virus-producing cells are not particularly limited as long as they are capable of producing a virus carrying the desired gene of interest, and mammalian cells from human, monkey, rodents, and the like, insect cells, and the like are used. Specific examples include HEK293 cells, MDCK cells, Vero cells, A549 cells, PerC6 cells, HeLa cells, CV-1 cells, LLC-MK2 cells, MDBK cells, WI-38 cells, MRC5 cells (human fibroblasts), BHK21 cells, insect cells, and the like, and HEK293 cells, MDCK cells, Vero cells, A549 cells, PerC6 cells, HeLa cells, insect cells, and the like are preferred, all of which being commercially available. When the virus is adenovirus or AAV, HEK293 cells, Vero cells, A549 cells, and the like are more preferably used, and HEK293 cells are further preferably used.

The method for introducing a gene of interest into a virus-producing cell is not particularly limited, and can be performed using gene transfer methods well known to those skilled in the art. More specifically, examples of such methods include chemical methods using transfection reagents such as cationic lipids, cationic polymers (e.g., polyetherimide (PEI), etc.), calcium phosphate, and the like; physical methods such as lipofection, electroporation, microinjection, sonoporation, laser irradiation, and the like; infection methods (biological methods) using viral vectors (e.g., adenovirus vector, adeno-associated virus vector, retrovirus vector, lentivirus vector, and Sendai virus vector); and the like. Chemical methods using transfection reagents are preferred, and chemical methods using cationic lipids or cationic polymers are more preferred. The gene of interest can be prepared by a method well known to those skilled in the art, but can also be obtained as a commercially available product.

Culturing can be either adherent culture or suspension culture, preferably suspension culture. In the present specification, the "adherent culture" refers to culturing cells by adhering them to a culture substrate, and specifically refers to a method in which cells are proliferated while being adhered to the surface of the culture substrate and while being adhered to each other. Examples of the culture substrate include, but are not limited to, multi-well plate, culture dish, Petri dish, culture flask, microcarrier, hollow fiber, and the like. Culturing may be static culture on a substrate. In the present specification, the "suspension culture" refers to a cell culture method performed in a state where cells do not adhere to the culture container. The suspension culture may or may not be accompanied by pressure from the outside or vibration on the liquid medium, or shaking or rotation operation in the liquid medium.

The culture vessel used for culturing is not particularly limited as long as it is capable of culturing the target cells, and examples include flask, tissue culture flask, dish, Petri dish, tissue culture dish, multi-dish, microplate, microwell plate, multi-plate, multi-well plate, microslide, chamber slide, schale, tube, tray, culture bag, roller bottle, bioreactor, and the like.

The culture vessel may be cell-adhesive or non-cell-adhesive, and is selected appropriately depending on the purpose. Cell-adhesive culture vessels may be coated with any cell-supporting substrate, such as an extracellular matrix (ECM), to improve adhesion of the surface of the culture vessel to cells. The cell-supporting substrate can be any substance intended for cell adhesion.

"Promoting the production of a virus carrying a gene of interest" refers to increasing the genomic titer of the gene of interest among all viruses produced during the culture of virus-producing mammalian cells into which the gene of interest has been introduced. That is, it refers to achieving (a) a state in which the full capsid ratio is improved and the overall genome titer is increased regardless of whether the capsid number (virus number) increases or decreases, or (b) a state in which the capsid number is increased and the overall genome titer is increased regardless of whether the full capsid ratio is high or low.

The medium composition 1 of the present invention may further contain other components that are favorable for virus production, as long as the effects of the present invention are not impaired. Other components include, for example, sugars such as glucose, fructose, sucrose, maltose, and the like; amino acids; proteins such as albumin, transferrin, and the like; peptides such as glycylglycylglycine, soybean peptide, and the like; serum; vitamins such as choline, vitamin A, B vitamins (thiamine, pyridoxine, cyanocobalamin, biotin, pantothenic acid, nicotinamide, and the like), vitamin C, vitamin E, and the like; fatty acids such as oleic acid, arachidonic acid, linoleic acid, and the like; lipids such as cholesterol and the like; inorganic salts such as sodium chloride, calcium chloride, sodium dihydrogen phosphate, and the like; trace elements such as zinc, selenium, and the like; buffers such as N,N-bis(2-hydroxyethyl)-2-aminoethanesulfonic acid (BES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukins, fibroblast growth factor (FGF), hepatocyte growth factor (HGF), transforming growth factor (TGF)-α, transforming growth factor (TGF)-β, vascular endothelial growth factor (VEGF), activin A, and the like; hormones such as dexamethasone, hydrocortisone, estradiol, progesterone, glucagon, insulin, and the like; and the like. Appropriate components can be selected and used depending on the type of cells to be cultured, the type of virus to be produced, and the like. The above-mentioned other components may be contained alone or in combination of two or more types thereof.

The other components can be contained in the medium composition 1 of the present invention at a total concentration of, for example, 0.001% to 99.9% by weight, preferably 0.01% to 99% by weight, more preferably 0.1% to 95% by weight.

Furthermore, the medium composition 1 of the present invention may contain serum or may be serum-free. Serum is not particularly limited as long as it is derived from an animal and does not inhibit the growth of cells. Preferred is a mammal-derived serum (e.g., fetal bovine serum, human serum, etc.), and more preferred is a fetal bovine serum. The concentration of the serum may be any as long as it is within a concentration range known per se. The medium composition 1 of the present invention is preferably serum-free.

In addition, the medium composition 1 of the present invention may or may not further contain β-alanine.

When the medium composition 1 of the present invention contains β-alanine, the concentration of β-alanine in the medium composition 1 of the present invention is generally 0.1 µM to 800 µM, preferably 1 µM to 700 µM, more preferably 3 µM to 600 µM, further preferably 10 µM to 500 µM, particularly preferably 30 µM to 400 µM.

The medium composition 1 of the present invention can be prepared, for example, by adding a PDH kinase inhibitor directly to a basal medium, and this embodiment is preferred because it is simple. Furthermore, when the PDH kinase inhibitor is in the form of a solution in the form of the medium additive 1 of the present invention described below, the medium composition 1 of the present invention can be obtained by adding, for example, 1/120 to 1/5, preferably 1/110 to 1/8, more preferably 1/100, of the amount of the medium additive 1 of the present invention to a basal medium.

The basal medium refers to a medium containing carbon sources, nitrogen sources, inorganic salts, and the like essential for culturing cells. It is not particularly limited as long as it achieves the effects of the present invention, and may be selected appropriately depending on the cells to be cultured.

Examples of the basal medium include Dulbecco's Modified Eagle's Medium (DMEM), Ham's Nutrient Mixture F12, DMEM/F12 medium, McCoy's 5A medium, Minimum Essential Medium (MEM), Eagle's Minimum Essential Medium (EMEM), alpha Modified Eagle's Minimum Essential Medium (αMEM), Roswell Park Memorial Institute (RPMI) 1640 medium, Iscove's Modified Dulbecco's Medium (IMDM), MCDB131 medium, William's Medium E, Fischer's Medium, mixture of these media, and the like. The basal medium may be prepared by a method known per se, or may be commercially available one, such as Viral Production Medium (Thermo Fisher Scientific: A4817901), HyClone HyCell TransFx-H transfection media (Cytiva: SH30939.01), BalanCD^{®} HEK293 (Fujifilm Wako Pure Chemical Industries, Ltd.: 551-34231), or EX-CELL^{®} 293 (Merck: 14571C), FreeStyle^{™} 293 Expression Medium (Thermo Fisher Scientific: 12338018), CDM4HEK293 (cytiva: SH30858.02), Expi293^{™} Expression Medium (Thermo Fisher Scientific: A1435101), and the like, or may be one currently under development.

When the medium composition 1 of the present invention contains the above-mentioned other components, serum, and/or β-alanine, it can be produced by adding a PDH kinase inhibitor and the above-mentioned other components, serum, and/or β-alanine to the above-mentioned basal medium as appropriate and mixing them directly, or by other production methods generally used in the field of medium compositions. The order of addition of each of these components is not particularly limited and can be determined appropriately depending on the intended use and the like.

The medium composition 1 of the present invention may be provided in a liquid state, or in a state of being concentrated more than the concentration at the time of use, or in a solid state such as freeze-dried powder and the like to be diluted with a solvent such as water and the like when in use, or dissolved or dispersed in a solvent such as water and the like before use.

By culturing virus-producing cells into which a gene of interest has been introduced, in the medium composition 1 of the present invention, the production of viruses carrying the gene of interest can be promoted regardless of whether or not it promotes the proliferation of the cells. In this sense, the medium composition 1 of the present invention can also be described as a "medium composition for promoting production of a virus carrying a gene of interest, regardless of the presence or absence of a proliferation promoting action on virus-producing cells into which the target gene has been introduced".

The present invention also provides use of a pyruvate dehydrogenase kinase inhibitor in the manufacture of a medium composition for promoting production of a virus carrying a gene of interest, when culturing a virus-producing cell into which the gene of interest has been introduced. Each term used and the like are as described above.

### 2. Medium additive 1

The present invention also provides a medium additive for promoting production of a virus carrying a gene of interest, when culturing a virus-producing cell into which the gene of interest has been introduced, the medium additive containing a pyruvate dehydrogenase kinase inhibitor (hereinafter also to be referred to as "the culture medium additive 1 of the present invention").

The "pyruvate dehydrogenase kinase inhibitor", "gene of interest", "virus", "virus-producing cell", "culturing", and "promoting production of a virus carrying a gene of interest", as well as the method for introducing a gene of interest into a virus-producing cell are as described above for the medium composition 1 of the present invention.

The concentration of the PDH kinase inhibitor in the medium additive 1 of the present invention is not particularly limited as long as the effects of the present invention are achieved. When the medium additive 1 is added to a basal medium and used, the PDH kinase inhibitor is contained in the medium additive 1 such that the final concentration in the basal medium is generally in the range of 0.1 mM to 1,000 mM, preferably 0.3 mM to 300 mM, more preferably 0.5 mM to 100 mM, further preferably 1 mM to 20 mM. For example, when the medium additive 1 of the present invention is in the form of a solution, the concentration of the PDH kinase inhibitor in the medium additive 1 is not particularly limited as long as the effects of the present invention are achieved, and is generally 1 mM to 10,000 mM, preferably 3 mM to 3,000 mM, more preferably 10 mM to 2,000 mM, further preferably 20 mM to 1,000 mM.

The "basal medium" is as described above for the medium composition 1 of the present invention.

The medium additive 1 of the present invention may contain components other than the PDH kinase inhibitor as long as the effects of the present invention are not impaired. Examples of other components include nutrients, vitamins, minerals, amino acids, sugars, and the like that are generally added to culture media. The above-mentioned other components may be contained alone, or in combination of two or more types thereof.

In addition, the medium additive 1 of the present invention may or may not contain β-alanine.

The "β-alanine" is as described above for the medium composition 1 of the present invention.

The content of the aforementioned other components and the like in the medium additive 1 of the present invention is not particularly limited, and the total amount is preferably 0.000001% to 99.99% by weight, more preferably 0.00001% to 99.9% by weight, further preferably 0.00001% to 99% by weight, further more preferably 0.0001% to 95% by weight, particularly preferably 0.0001% to 90% by weight in the medium additive 1 of the present invention.

When the other component is β-alanine and the medium additive 1 is added to a basal medium and used, β-alanine is contained in the medium additive 1 such that the final concentration in the basal medium is generally in the range of 0.1 µM to 800 µM, preferably 1 µM to 700 µM, more preferably 3 µM to 600 µM, further preferably 10 µM to 500 µM, particularly preferably 30 µM to 400 µM. For example, when the medium additive 1 of the present invention is in the form of a solution, the concentration of β-alanine in the medium additive 1 is generally 1 µM to 500 mM, preferably 10 µM to 475 mM, more preferably 30 µM to 450 mM, further preferably 100 µM to 425 mM, particularly preferably 300 µM to 400 mM.

When the medium additive 1 of the present invention is a PDH kinase inhibitor itself, it can be used as is. When it contains the above-mentioned other component and the like, it can be produced by adding the above-mentioned other component and the like to the PDH kinase inhibitor as appropriate and mixing them directly, or by other production methods generally used in the field of the medium additive 1. The order of addition of each of these components is not particularly limited and can be determined appropriately depending on the intended use and the like. The medium additive 1 of the present invention may be in the form of a solution, or may be made into a solid powder by lyophilizing the solution, or the like.

The medium additive 1 of the present invention can generally be used by adding same to a basal medium. In addition, for example, when mammalian cells that produce a virus are cultured in a medium that does not contain a pyruvate dehydrogenase kinase inhibitor and proliferated to a certain extent, the number of cells is adjusted as necessary, a gene of interest is introduced into the cells, and the cells are subsequently cultured in the same medium, the medium additive 1 of the present invention can be added to the above-mentioned medium before (e.g., several hours before), during, or after (e.g., several hours after) introducing the gene of interest into the virus-producing mammalian cells. For example, when the medium additive 1 of the present invention is a solution, it is used by adding a 1/120 to 1/5, preferably 1/110 to 1/8, more preferably 1/100, of the amount of the medium additive 1 to a basal medium or the above-mentioned medium not containing a pyruvate dehydrogenase kinase inhibitor.

By culturing virus-producing cells into which a gene of interest has been introduced, in a medium obtained by adding the medium composition 1 of the present invention to a basal medium or the above-mentioned medium not containing a pyruvate dehydrogenase kinase inhibitor, the production of viruses carrying the gene of interest can be promoted regardless of whether or not it promotes the proliferation of the cells. In this sense, the medium additive 1 of the present invention can also be described as a "medium additive for promoting production of a virus carrying a gene of interest, regardless of the presence or absence of a proliferation promoting action on virus-producing cells into which the target gene has been introduced".

### 3. Method for promoting production of virus carrying gene of interest

The present invention also provides a method for promoting production of a virus carrying a gene of interest, including a step of culturing a virus-producing cell into which the gene of interest has been introduced, in a medium containing a pyruvate dehydrogenase kinase inhibitor (hereinafter also to be referred to as "the promoting method 1 of the present invention").

The "pyruvate dehydrogenase kinase inhibitor", "gene of interest", "virus", "virus-producing cell", "culturing", and "promoting production of a virus carrying a gene of interest", as well as the method for introducing a gene of interest into a virus-producing cell are as described above for the medium composition 1 of the present invention. The "medium containing a pyruvate dehydrogenase kinase inhibitor" refers to a medium having a composition similar to that of the medium composition 1 of the present invention.

A virus-producing cell into which the gene of interest has been introduced, can be cultured under general culture conditions used when culturing animal cells. For example, culturing at 95% humidity and a CO₂ concentration of 5%-10% (v/v) is exemplified, but is not limited to these conditions. Culturing can be performed, for example, at 30°C to 37°C, or may also be performed at temperatures outside the aforementioned range as long as proliferation of the desired cell and production of a virus into which a gene of interest has been introduced can be achieved. The culture period is not particularly limited and is generally 1 hr to 14 days, preferably 5 hr to 7 days, more preferably 12 hr to 150 hr, further more preferably 48 hr to 120 hr.

In Promotion Method 1 of the present invention, culturing of virus-producing cells into which a gene of interest has been introduced can be performed by any of the following methods.
(1) Virus-producing cells are cultured in a medium containing a pyruvate dehydrogenase kinase inhibitor and proliferated to a certain extent, the number of cells is adjusted as necessary, a gene of interest is introduced into the cells, and the cells are subsequently cultured in the same medium.
(2) Virus-producing cells are cultured in a medium that does not contain a pyruvate dehydrogenase kinase inhibitor and proliferated to a certain extent, the number of cells is adjusted as necessary, the medium is replaced with a medium containing a pyruvate dehydrogenase kinase inhibitor, then a gene of interest is introduced into the cells, and the cells are subsequently cultured in the same medium (the order of medium replacement and introduction of the gene of interest may be reversed).
(3) Virus-producing cells are cultured in a medium that does not contain a pyruvate dehydrogenase kinase inhibitor and proliferated to a certain extent, the number of cells is adjusted as necessary, a gene of interest is introduced into the cells, and the cells are subsequently cultured in the same medium. Here, a pyruvate dehydrogenase kinase inhibitor is added to the above-mentioned medium before (e.g., several hours before), during, or after (e.g., several hours after) introducing the gene of interest into the virus-producing cells.

By the promoting method 1 of the present invention, the production of a virus carrying a gene of interest can be promoted regardless of whether or not it promotes the proliferation of a virus-producing cell into which the gene of interest has been introduced.

### 4. Method for producing virus carrying gene of interest

The present invention also provides a method for producing a virus carrying a gene of interest, including a step of culturing a virus-producing cell into which the gene of interest has been introduced, in a medium containing a pyruvate dehydrogenase kinase inhibitor (hereinafter also to be referred to as "the production method 1 of the present invention").

The "pyruvate dehydrogenase kinase inhibitor", "gene of interest", "virus", "virus-producing cell", and "culturing", as well as the method for introducing a gene of interest into a virus-producing cell are as described above for the medium composition 1 of the present invention. The "culturing" method is as described above for the promoting method 1 of the present invention. In addition, the "medium containing a pyruvate dehydrogenase kinase inhibitor" refers to a medium having a composition similar to that of the medium composition 1 of the present invention.

In the production method 1 of the present invention, after culturing virus-producing cells into which a gene of interest has been introduced, in a medium containing a pyruvate dehydrogenase kinase inhibitor, the virus carrying the gene of interest can be purified by a method known per se. For example, a virus carrying a gene of interest can be purified by recovering cultured cells from the obtained culture solution, disrupting the cells, and subjecting the obtained cell lysate containing the virus to appropriate steps such as filtration, ultracentrifugation, chromatography, and ultrafiltration.

By the production method 1 of the present invention, the production of a virus carrying a gene of interest can be promoted regardless of whether or not it promotes the proliferation of a virus-producing cell into which the gene of interest has been introduced.

### 5. Medium composition 2

The present invention also provides a medium composition for promoting production of a virus or virus-like particle when culturing a cell that produces the virus or virus-like particle, the medium composition containing a pyruvate dehydrogenase kinase inhibitor (hereinafter also to be referred to as "the medium composition 2 of the present invention").

The "pyruvate dehydrogenase kinase inhibitor", "virus", and "culture" are as described above for the medium composition 1 of the present invention.

A virus-like particle refers to a molecule that resembles a virus but does not contain genetic substance and is not infectious.

As the virus or virus-like particle, a virus is preferred.

The cell that produces virus or virus-like particle is similar to the "virus-producing cell" described above for the medium composition 1 of the present invention.

As long as the effects of the present invention are not impaired, the medium composition 2 of the present invention may contain other components, serum, and/or β-alanine similar to those described above for the medium composition 1 of the present invention, in the contents similar to those described above for the medium composition 1 of the present invention.

The concentration of the pyruvate dehydrogenase kinase inhibitor in the medium composition 2 of the present invention, and the production method, form, and method of use of the medium composition, are as described above for the medium composition 1 of the present invention.

By culturing cells that produce virus or virus-like particle in the medium composition 2 of the present invention, the production of the virus or virus-like particle can be promoted even when an action that promotes proliferation of the cells is not afforded. In this sense, the medium composition 2 of the present invention can also be referred to as a "medium composition for promoting production of virus or virus-like particle, regardless of the presence or absence of a proliferation promoting action on cells that produce virus or virus-like particle".

The present invention also provides use of a pyruvate dehydrogenase kinase inhibitor in the manufacture of a medium composition for promoting production of a virus or virus-like particle, when culturing a cell that produces the virus or virus-like particle.

### 6. Medium additive 2

The present invention also provides a medium additive for promoting production of a virus or virus-like particle when culturing a cell that produces the virus or virus-like particle, the medium additive comprising a pyruvate dehydrogenase kinase inhibitor (hereinafter also to be referred to as "the medium additive 2 of the present invention").

The "pyruvate dehydrogenase kinase inhibitor", "virus", and "culturing" are as described above for the medium composition 1 of the present invention, and the "virus-like particle" is as described above for the medium composition 2 of the present invention.

As the virus or virus-like particle, a virus is preferred.

The cell that produces virus or virus-like particle is similar to the "virus-producing cell" described above for the medium composition 1 of the present invention.

As long as the effects of the present invention are not impaired, the medium additive 2 of the present invention may contain components other than PDH kinase inhibitor similar to those described above for the medium additive 1 of the present invention, in the contents similar to those described above for the medium additive 1 of the present invention.

The concentration of the PDH kinase inhibitor in the medium additive 2 of the present invention, and the production method, form, and method of use of the medium additive, are as described above for the medium additive 1 of the present invention.

By culturing cells that produce virus or virus-like particle, by adding the medium additive 2 of the present invention to a basal medium, the production of the virus or virus-like particle can be promoted even when an action that promotes proliferation of the cells is not afforded. In this sense, the medium additive 2 of the present invention can also be referred to as a "medium additive for promoting production of virus or virus-like particle, regardless of the presence or absence of a proliferation promoting action on cells that produce virus or virus-like particle".

The "basal medium" is as described above for the medium composition 1 of the present invention.

### 7. Method for promoting production of virus or virus-like particle

The present invention also provides a method for promoting production of a virus or virus-like particle, including a step of culturing a cell that produces the virus or virus-like particle in a medium containing a pyruvate dehydrogenase kinase inhibitor (hereinafter also to be referred to as "the promoting method 2 of the present invention").

The "pyruvate dehydrogenase kinase inhibitor", "virus", and "culturing" are as described above for the medium composition 1 of the present invention, and the "virus-like particle" is as described above for the medium composition 2 of the present invention. In addition, the "medium containing a pyruvate dehydrogenase kinase inhibitor" refers to a medium having a composition similar to that of the medium composition 1 of the present invention.

As the virus or virus-like particle, a virus is preferred.

The cell that produces virus or virus-like particle is similar to the "virus-producing cell" described above for the medium composition 1 of the present invention.

A cell that produces virus or virus-like particle can be cultured under general culture conditions used when culturing animal cells. For example, culturing at 95% humidity and a CO₂ concentration of 5%-10% (v/v) is exemplified, but is not limited to these conditions. Culturing can be performed, for example, at 30°C to 37°C, or may also be performed at temperatures outside the aforementioned range as long as proliferation of the desired cell and production of a virus or virus-like particle can be achieved. The culture period is not particularly limited and is generally 1 hr to 14 days, preferably 5 hr to 7 days, more preferably 12 hr to 150 hr, further more preferably 48 hr to 120 hr. Furthermore, a PDH kinase inhibitor may be added at some time point during the culturing and the culturing may be continued.

According to the culture method 2 of the present invention, production of virus or virus-like particle can be promoted even when an action that promotes proliferation of the virus or virus-like particle is not afforded.

### 8. Method for producing virus or virus-like particle

The present invention also provides a method for producing a virus or virus-like particle, including a step of culturing a cell that produces the virus or virus-like particle in a medium containing a pyruvate dehydrogenase kinase inhibitor (hereinafter also to be referred to as "the production method 2 of the present invention").

The "pyruvate dehydrogenase kinase inhibitor", "virus", and "culturing" are as describedabove for the medium composition 1 of the present invention, and the "virus-like particle" is as described above for the medium composition 2 of the present invention. The "culturing" method is as described above for the promoting method 2 of the present invention. In addition, the "medium containing a pyruvate dehydrogenase kinase inhibitor" refers to a medium having a composition similar to that of the medium composition 1 of the present invention.

As the virus or virus-like particle, a virus is preferred.

The cell that produces virus or virus-like particle is similar to the "virus-producing cell" described above for the medium composition 1 of the present invention.

In the production method 2 of the present invention, after culturing cells that produce virus or virus-like particle in a medium containing a pyruvate dehydrogenase kinase inhibitor, the virus or virus-like particle can be purified by a method known per se. For example, a virus or virus-like particle can be purified by recovering cultured cells from the obtained culture solution, disrupting the cells, and subjecting the obtained cell lysate containing the virus or virus-like particle to appropriate steps such as filtration, ultracentrifugation, chromatography, and ultrafiltration.

According to the production method 2 of the present invention, production of virus or virus-like particle can be promoted even when an action to promote proliferation of cells that produce the virus or virus-like particle is not afforded.

The present invention is now described in more detail by referring to Examples and the like, but the present invention is not limited to these examples in any way.

### [Example]

In the following Example 1, the ability of human embryonic kidney cell 293 (HEK293) to produce AAV was evaluated using sodium dichloroacetate. The reagents, cells, and medium used are as follows:
Sodium dichloroacetate: Sodium dichloroacetate (FUJIFILM Wako Pure Chemical Industries, Ltd., 326-87772)
HEK293: Viral Production Cells 2.0 (Thermo Fisher Scientific, A49784)
medium for HEK293: Viral Production Medium (Thermo Fisher Scientific: A4817901)

### Example 1: Production of AAV in HEK293 by adding sodium dichloroacetate in suspension culture system

Viral Production Cells 2.0 were seeded in 30 mL of Viral Production Medium supplemented with GlutaMAX^{™} Supplement (Thermo Fisher Scientific: 35050061) to a final concentration of 4 mM in a 125 mL Erlenmeyer flask (VIOLAMO, SEF125V). The cells were then cultured in an incubator set at 37°C, 8% CO₂ under stirring for two passages. The cells were then seeded at a cell density of 0.6×10⁶ cells/mL in 30 mL of Viral Production Medium supplemented with GlutaMAX^{™} Supplement to a final concentration of 4 mM, or in 30 mL of Viral Production Medium supplemented with GlutaMAX^{™} Supplement to a final concentration of 4 mM and sodium dichloroacetate to a final concentration of 5 mM, and cultured under stirring for 3 days. On day 3, the number of viable cells was measured using a viable cell autoanalyzer Vi-CELL BLU (manufactured by Becman). Then, the cells were diluted with a fresh medium to a viable cell count of 3×10⁶ cells/mL, and 3 mL thereof was seeded in a 6-well suspension culture plate (SUMITOMO BAKELITE CO., LTD.: MS-8006R). Thereafter, using a cationic lipid-based AAV-MAX Transfection Kit (manufactured by Thermo Fisher Scientific: A50515), AAVpro^{®} Packaging Plasmid (AAV8) (Takara Bio Inc.: 6681) and pAAV-ZsGreen1 Vector (Takara Bio Inc.: 6231) were transfected. After 3 days of culturing, AAV8 vectors were extracted using AAVpro^{®} Titration Kit (for Real Time PCR) Ver.2 (Takara Bio: 6233), and the AAV8 genome titer was measured using 7500 Fast Real-Time PCR System (Thermo Fisher Scientific: 4357362). In addition, the number of viral particles was also measured using AAV-8 Titration ELISA Kit (PROGEN Biotechnik: PRAAV8). The full/empty capsid ratio (full capsid ratio) was calculated therefrom.

The influence of sodium dichloroacetate on HEK293 proliferation and AAV production was examined in a series of verification, and the results are shown in Figs. 1 to 3. The viable cell density before dilution on day 3 after seeding is shown in Fig. 1, the AAV8 genome titer (relative) is shown in Fig. 2, and the full capsid ratio (relative) is shown in Fig. 3. The addition of sodium dichloroacetate slightly inhibited cell proliferation, but improvements in the AAV8 genome titer and full capsid ratio were confirmed.

### Example 2: Production of adenovirus in HEK293 by adding sodium dichloroacetate in suspension culture system

In the following Example, the ability of human embryonic kidney cell 293 (HEK293) to produce adenovirus can be evaluated using sodium dichloroacetate. The reagents, cells, and media to be used are as follows:
Sodium dichloroacetate: sodium dichloroacetate (FUJIFILM Wako Pure Chemical Corporation, 326-87772)
HEK293: suspension type HEK293 2. sus (ATCC: CRL-1573.3) Adenovirus: Human adenovirus 32 (ATCC V R-625^{™})
media for HEK293: 293 SFM II (Thermo Fisher Scientific), CDM4HEK293 (Cytiva)

(1) Frozen cells are thawed in a thermostatic tank set to about 37°C, 10 mL of glutamine-containing 293 SFM II is charged in a 15 mL centrifuge tube, and 1 mL of thawed cell solution is added thereto and the cells are suspended.
(2) Centrifugation is performed for 5 min (1000 rpm, 190×g, 24°C) using a centrifuge (AX-310, Tomy Seiko Co., Ltd.) and the supernatant is removed.
(3) 1 mL of passage medium is added to a 15 mL centrifuge tube and the cells are resuspended. The entire volume is placed in a 50 mL tube, 29 mL of glutamine-containing 293 SFM II is added thereto and the cells are suspended. 5 mL of the cell suspension is placed in a Petri dish and culturing with shaking (50 rpm) is performed in a CO₂ incubator (temperature setting: 37°C, CO₂ concentration: 5%, model: MCO-170AICUV-PJ, Panasonic Healthcare Co., Ltd.) for 2-3 days.
(4) After culturing, the culture solution is recovered and transferred to a 15 mL centrifuge tube.
(5) Centrifugation is performed for 5 min (1000 rpm, 190×g, 24°C) and the supernatant is removed.
(6) 1 mL of glutamine-containing 293 SFM II is added to the 15 mL centrifuge tube and the cells are resuspended. The cell number is measured using a hemocytometer and glutamine-containing 293 SFM II is added to 3×10⁵ cells/mL. 5 mL of the adjusted cell suspension is added to Petri dish and culturing with shaking (50 rpm) is performed in a CO₂ incubator for 3-4 days.
(7) After culturing, steps (4) to (6) are repeated once.
(8) After culturing in a CO₂ incubator for 3-4 days, the culture solution is recovered and transferred to a 15 mL centrifuge tube.
(9) Centrifugation is performed for 5 min (1000 rpm, 190×g, 24°C) and the supernatant is removed.
(10) 1 mL of a fresh culture solution is added to the 15 mL centrifuge tube and the cells are resuspended. The cell number is measured using a hemocytometer and the culture solution is added to 3×10⁵ cells/mL. 5 mL of the adjusted cell suspension is added to two Petri dishes and culturing with shaking (50 rpm) is performed in a CO₂ incubator for 3-4 days.
(11) After culturing, steps (8) to (10) are repeated three times.
(12) After culturing for 3 days, the cell number is measured (Fig. 1) and adjusted to 1×10⁵ cells/mL using CDM4HEK293 with or without addition of sodium dichloroacetate, and 5 mL thereof is added per Petri dish. The frozen virus solution is thawed and added to the Petri dish at an MOI of 0.1 for 1×10⁵ cells/mL.

Improvement in the adenovirus titer by the addition of sodium dichloroacetate can be confirmed.

### [Industrial Applicability]

The present invention makes it possible to improve the production yield of a virus carrying a gene of interest when producing the virus carrying the gene of interest using virus-producing cells, and the like.

This application is based on a patent application No. 2023-143946 filed in Japan (filing date: September 5, 2023), the contents of which are incorporated in full herein.

## Claims

1. A medium composition for promoting production of a virus carrying a gene of interest for culturing a virus-producing cell into which the gene of interest has been introduced, the medium composition comprising a pyruvate dehydrogenase kinase inhibitor.

2. The medium composition according to claim 1, wherein the pyruvate dehydrogenase kinase inhibitor is dichloroacetic acid or a salt thereof.

3. The medium composition according to claim 1, wherein the cell is a cell selected from the group consisting of a HEK293 cell, an MDCK cell, a Vero cell, an A549 cell, a PerC6 cell, a HeLa cell, and an insect cell.

4. The medium composition according to claim 1, wherein the cell is an HEK293 cell.

5. The medium composition according to claim 1, wherein the virus-producing cell is a cell that produces adeno-associated virus.

6. The medium composition according to any one of claims 1 to 5, wherein a concentration of the pyruvate dehydrogenase kinase inhibitor in the medium composition is 0.1 mM to 1,000 mM.

7. A medium additive for promoting production of a virus carrying a gene of interest, when culturing a virus-producing cell into which the gene of interest has been introduced, the medium additive comprising a pyruvate dehydrogenase kinase inhibitor.

8. Use of a pyruvate dehydrogenase kinase inhibitor in the manufacture of a medium composition for promoting production of a virus carrying a gene of interest, when culturing a virus-producing cell into which the gene of interest has been introduced.

9. A method for promoting production of a virus carrying a gene of interest, comprising a step of culturing a virus-producing cell into which the gene of interest has been introduced, in a medium comprising a pyruvate dehydrogenase kinase inhibitor.

10. A method for producing a virus carrying a gene of interest, comprising a step of culturing a virus-producing cell into which the gene of interest has been introduced, in a medium comprising a pyruvate dehydrogenase kinase inhibitor.

11. A medium composition for promoting production of a virus or virus-like particle when culturing a cell that produces the virus or virus-like particle, the medium composition comprising a pyruvate dehydrogenase kinase inhibitor.

12. The medium composition according to claim 11, wherein the pyruvate dehydrogenase kinase inhibitor is dichloroacetic acid or a salt thereof.

13. The medium composition according to claim 11, wherein the cell is a cell selected from the group consisting of a HEK293 cell, an MDCK cell, a Vero cell, an A549 cell, a PerC6 cell, an HeLa cell, and an insect cell.

14. The medium composition according to claim 11, wherein the cell is an HEK293 cell.

15. The medium composition according to claim 11, wherein the cell that produces the virus or virus-like particle is a cell that produces adeno-associated virus.

16. The medium composition according to any one of claims 11 to 15, wherein a concentration of the pyruvate dehydrogenase kinase inhibitor in the medium composition is 0.1 mM to 1,000 mM.

17. A medium additive for promoting production of a virus or virus-like particle when culturing a cell that produces the virus or virus-like particle, the medium additive comprising a pyruvate dehydrogenase kinase inhibitor.

18. Use of a pyruvate dehydrogenase kinase inhibitor in the manufacture of a medium composition for promoting production of a virus or virus-like particle, when culturing a cell that produces the virus or virus-like particle.

19. A method for promoting production of a virus or virus-like particle, comprising a step of culturing a cell that produces the virus or virus-like particle in a medium comprising a pyruvate dehydrogenase kinase inhibitor.

20. A method for producing a virus or virus-like particle, comprising a step of culturing a cell that produces the virus or virus-like particle in a medium comprising a pyruvate dehydrogenase kinase inhibitor.
